Europäisches Patentamt

European Patent Office

Office européen des brevets

(11) Veröffentlichungsnummer: **0 247 573**
**A2**

(19)

(12) # EUROPÄISCHE PATENTANMELDUNG

(21) Anmeldenummer: 87107634.5

(22) Anmeldetag: 26.05.87

(51) Int. Cl.⁴: **C07D 253/08** , C07K 1/08

Die Bezeichnung der Erfindung wurde geändert (Richtlinien für die Prüfung im EPA, A-III, 7.3).

(30) Priorität: 30.05.86 DE 3618218

(43) Veröffentlichungstag der Anmeldung:
02.12.87 Patentblatt 87/49

(84) Benannte Vertragsstaaten:
AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: König, Wolfgang, Dr.
Eppsteiner Strasse 25
D-6238 Hofheim am Taunus(DE)
Erfinder: Knolle, Jochen, Dr.
Höchster Strasse 21
D-6239 Kriftel(DE)
Erfinder: Breipohl, Gerhard, Dr.
Geisenheimer Strasse 95
D-6000 Frankfurt am Main(DE)

(54) **Aminosäure-Benzotriazinylester, Verfahren zu deren Herstellung und deren Verwendung.**

(57) Die Erfindung betrifft Aminosäurederivate der Formel
X-A-OObt
in welcher X eine Urethanschutzgruppe, A einen gegebenenfalls geschützten Rest einer D-oder L-Aminosäure und OObt 4-Oxo-3,4-dihydro-1,2,3-benzotriazin-3-yloxy bedeuten, Verfahren zu deren Herstellung und deren Verwendung.

EP 0 247 573 A2

## Aminosäure-OObt-Ester, Verfahren zu deren Herstellung und deren Verwendung

Für die Synthese von Peptiden nach der klassischen Lösungsmittelmethodik insbesondere aber nach der Festphasenmethode (B. Merrifield, Adv. Enzymol. 32[1969] 221-296) ist es günstig bei der Kupplungsreaktion statt der in situ Aktivierung mit einem Aktivierungsreagenz, wie z.B. Carbodiimiden, schon eine aktivierte Spezies wie symetrische Anhydride oder Aktivester einzusetzen. Dadurch wird insbesondere bei der Festphasensynthese der Kontakt der reaktiven Aminogruppe der an das Trägerharz gebundenen Aminosäure mit dem Aktivierungsreagenz vermieden. Die Verwendung der symmetrischen Anhydride hat jedoch den Nachteil, daß diese in vielen Fällen nicht isolierbar sind und man zum anderen von vornherein die doppelte Menge an Aminosäurederivat einsetzen muß. Lange bekannt ist die Verwendung von N-Hydroxysuccinimid-, Trichlorphenyl-oder Nitrophenylestern.

Die Hydroxysuccinimidester sind für die Festphasensynthese nicht reaktiv genug, was zu sehr langen Cycluszeiten führt, die Trichlorphenylester sind wegen des Dioxinprolems umstritten und die Nitrophenylester sind häufig nicht gut isolierbar und racemisierungssicher.

In neuerer Zeit wurden auch häufig die Pentafluorphenylester eingesetzt, diese sind jedoch zum Teil schlecht zugänglich oder schwierig zu reinigen. Bei der Anwendung in der Festphasensynthese ist außerdem zum Erreichen einer schnellen Reaktion der Zusatz von HOBt unerläßlich.

Es wurden neue HOObt-Ester gefunden, die sich ausgezeichnet zur Peptidsynthese durch Fragmentkupplung insbesondere mittels der Festphasenmethode eignen.

Die Erfindung betrifft daher Aminosäurederivate der Formel

X - A - OObt

in welcher

X eine Urethanschutzgruppe,

A Gly, Ala, Val, Leu, Ile, Cys(tBu), Cys(Acm), Met, Pro, Lys(Boc), Asp(OtBu), Glu(OtBu), Phe, Tyr-(tBu), Trp, Lys(Z), Lys(Bzl) oder Cys (4-MeOBzl), jeweils in ihrer L-oder ihrer D-Form und

OObt 4-Oxo-3,4-dihydro-1,2,3-benzotriazin-3-yloxy bedeuten.

Bevorzugt ist die reine Form dieser Ester.

Die hier beschriebenen neuen HOObt-Ester bieten den Vorteil, daß sie sehr rein zu erhalten und gut lagerstabil sind. Das bei der Reaktion freigesetzte HOObt kann aufgrund seiner Farbe zudem als Indikator für das Fortschreiten der Reaktion herangezogen werden. Weiterhin sind die hier beschriebenen Ester in den für die Festphasensynthese herangezogenen Lösungsmitteln (Dimethylformamid (DMF), Dimethylacetamid (DMA), N-Methylpyrrolidon (NMP)) gut löslich im Gegensatz zu den freien Fmoc-Aminosäuren, was vorteilhaft ihre Verwendung in Peptidsyntheseautomaten ermöglicht.

Geeignete Urethanschutzgruppen sind bei Hubbuch, Kontakte Merck 1979 Nr. 3, Seite 11 ff. beschrieben. Bevorzugt sind Fmoc und Boc, insbesondere aber Fmoc.

Die Erfindung betrifft auch ein Verfahren zur Herstellung von Verbindungen der Formel X-A-OObt, das dadurch gekennzeichnet ist,
daß man Verbindungen der Formel X-A-OH, worin X und A wie oben definiert sind, mit 3-Hydroxy-4-oxo-3,4-dihydro-1,2,3-benzotriazin (HOObt) in Gegenwart eines Carbodiimids umsetzt. Als Reaktionsmedium sind inerte Lösungsmittel, wie THF, DMF, Chloroform, Methylenchlorid, Acetonitril oder deren Gemische geeignet; bevorzugt ist Ether wie THF. Man führt die Umsetzung bei -20 bis +50°C, vorzugsweise bei -10°C bis etwa Raumtemperatur durch.

Die Erfindung betrifft weiterhin die Verwendung der neuen Aminosäurederivate der Formel X-A-OObt bei der Herstellung von Peptiden mittels Fragmentkupplung, insbesondere bei der Festkörpersynthese.

Bei der Fragmentkupplung in Lösung wird ein gegebenenfalls geschütztes Fragment in Gegenwart von HOObt mit einer Verbindung der Formel X-A-OObt in einem geeigneten inerten Lösungsmittel bei -20 bix +50°C, vorzugsweise bei 0°C bis Raumtemperatur umgesetzt. Geeignet sind beispielsweise die oben genannten Lösungsmittel, vorzugsweise DMF. Argininhaltige Fragmente können in Form ihrer besser löslichen Perchlorate eingesetzt werden, die zweckmäßig durch Zugabe von Pyridiumperchlorat zum Reaktionsgemisch in situ hergestellt werden.

Nach beendeter Reaktion werden gewünschtenfalls temporär eingeführte Schutzgruppen in an sich bekannter Weise abgespalten.

Die Festphasensynthese beginnt am C-terminalen Ende des Peptids mit der Kupplung einer geschützten Aminosäure an ein entsprechendes Harz. Derartige Ausgangsmaterialien können durch Verknüpfung einer geschützten Aminosäure mit einem mit einer Chlormethyl-, Hydroxymethyl-, Benzhydrylamino(BHA)-, Methylbenzhydrylamino-(MBHA)-Gruppe modifiziertem Polystyrol-oder Polyacrylamid-Harz über eine Ester-bzw. Amidbindung erhalten werden. Die als Trägermaterial verwendeten Harze sind kommerziell erhältlich. BHA-und MBHA-Harze werden für gewöhnlich verwendet, wenn das synthetisierte Peptid am C-Terminus eine freie Amidgruppe enthalten soll. Falls das Peptid eine sekundäre Amidgruppe am C-terminalen Ende enthalten soll wird ein Chlormethyl-bzw. Hydroxymethyl-Harz verwendet und die Abspaltung mit den entsprechenden Amin durchgeführt. Sollen im Peptid die tert.-Butyl-Schutzgruppen der Aminosäure Seitenkette erhalten bleiben, so wird die Synthese mit der Fmoc-Schutzgruppe zur temporären Blockierung der α-Amino-Gruppe der Aminosäure unter Verwendung der z.B. bei R.C. Sheppard, J.Chem.Soc., Chem.Comm. 1982, 587 beschriebenen Methodik durchgeführt, wobei die Guanidino-Funktion des Arginins vorzugsweise durch Protonierung mit Pyridinium-Perchlorat geschützt wird und der Schutz der anderen in der Seitenkette funktionalisierten Aminosäuren mit durch katalytische Transferhydrierung (A. Felix et al. J. Org. Chem. 13, 4194 (1978) oder mit Natrium in flüssigem Ammoniak ( W.Roberts, J.Am.Chem.Soc. 76, 6203 (1954) abspaltbaren Benzylschutzgruppen erfolgt.

Nach Abspaltung der Aminoschutzgruppen der an das Harz gekuppelten Aminosäure mit einem geeigneten Reagenz, wie z.B. Trifluoressigsäure in Methylenchlorid im Falle der Boc-Schutzgruppe oder einer 20 %igen Lösung von Piperidin in Dimethylformamid im Falle der Fmoc-Schutzgruppe, werden die nachfolgend geschützten Aminosäuren nacheinander in der gewünschten Reihenfolge aufgekuppelt.
Die intermediär entstehenden N-terminal geschützten Peptidharze werden vor der Verknüpfung mit dem nachfolgenden Aminosäurederivat durch die vorbeschriebenen Reagenzien entblockiert.

Zur Voraktivierung von Aminosäurederivaten in situ können alle möglichen in der Peptidsynthese verwendeten Aktivierungs-Reagenzien, siehe z.B. Houben-Weyl, Methoden der organischen Chemie, Band 15/2, verwendet werden, insbesondere aber Carbodiimide wie z.B. N,N'-Dicyclohexylcarbodiimid, N,N'-Diisopropylcarbodiimid oder N-Ethyl-N'-(3-dimethylaminopropyl)carbodiimid. Die Kupplung kann dabei direkt durch Addition von einem Aminosäurederivat der Formel X-A-OObt oder eines in situ voraktivierten Aminosäurederivats und gegebenenfalls eines die Racemisierung unterdrückenden Zusatzes wie z.B. 1-Hydroxybenzotriazol (HOBt) (W. König, R. Geiger, Chem. Ber. 103, 708 (1970) oder 3-Hydroxy-4oxo-3.4-dihydrobenzotriazin (HOObt) (W. König, R. Geiger, Chem. Ber. 103, 2054 (1970) zum Harz durchgeführt werden.

Die Kupplung bzw. Aktivierung der Aminosäurederivate mit einem der oben genannten Aktivierungsreagenzien kann in den oben genannten Lösungsmitteln vorzugsweise in Dimethylformamid oder Methylenchlorid oder einer Mischung aus beiden durchgeführt werden. Die Temperatur beträgt -20° bis +50°C, vorzugsweise 0°C bis etwa Raumtemperatur. Das aktivierte Aminosäurederivat wird üblicherweise in einem 1,5 bis 4 fachen Überschuß eingesetzt. In Fällen, in denen eine unvollständige Kupplung eintritt, wird die Kupplungsreaktion wiederholt, ohne vorher die für die Kupplung der nächstfolgenden Aminosäure nötige Entblockierung der α-Aminogruppe des Peptidharzes durchzuführen

Der erfolgreiche Ablauf der Kupplungsreaktion kann beispielsweise mittels der Ninhydrin-Reaktion, wie z.B. von E. Kaiser et al. Anal. Biochem. 34 595 (1970) beschrieben, überprüft werden. Die Synthese kann auch automatisiert z.B. mit einem Peptid-Synthesizer Modell 430A der Fa. Applied Biosystems durchgeführt werden, wobei entweder die vom Gerätehersteller vorgesehenen Syntheseprogramme oder aber auch vom Benutzer selbst erstellte verwendet werden können. Letztere werden insbesondere bei der Verwendung von mit der Fmoc-Gruppe geschützten Aminosäurederivaten eingesetzt.

Nach Synthese der Peptide in der vorgehend beschriebenen Weise kann das Peptid vom Harz mit Reagenzien, wie z.B. flüssigem Fluorwasserstoff (bevorzugt bei den nach der Boc-Methode hergestellten Peptiden) oder Trifluoressigsäure (bevorzugt bei den nach der Fmoc-Methode synthetisierten Peptiden) abgespalten werden. Diese Reagenzien spalten nicht nur das Peptid vom Harz sondern auch die weiteren Seitenkettenschutzgruppen der Aminosäurederivate. Auf diese Weise erhält man außer bei der Verwendung von BHA-und MBHA-Harzen das Peptid in Form der freien Säure. Bei den BHA-bzw. MBHA-Harzen erhält man bei der Spaltung mit Fluorwasserstoff das Peptid als Säureamid. Will man z.B. das Ethylamid erhalten, kann das Peptid mit Ethylamin vom Harz abgespalten werden, wobei die Abspaltung der Seitenketten-Schutzgruppen nachfolgend durch andere geeignete Reagenzien, wie z.B. die oben genannten erfolgt.

Bei der Abspaltung mit Fluorwasserstoff und Trifluoressigsäure werden als Kationenfänger üblicherweise Substanzen, wie Phenol, Kresol, Thiokresol, Thioanisol, Dimethylsulfid, Ethylmethylsulfid oder eine Mischung von zwei oder mehr dieser Hilfsmittel zugesetzt. Die Trifluoressigsäure kann dabei auch durch geeignete Lösungsmittel, wie z.B. Methylenchlorid verdünnt angewendet werden. Falls die tert.-Butyl bzw. Benzylseitenketten-Schutzgruppen der Peptide erhalten bleiben sollen, wird die Abspaltung des an einem besonders modifizierten Trägerharz synthetisierten Peptides mit 1 % Trifluoressigsäure in Methylenchlorid durchgeführt, wie z.B. bei R.C. Sheppard J.Chem.Soc., Chem.Comm. 1982 , 587 beschrieben. Sollen ein-

zelne tert.-Butyl bzw. Benzylseitenketten-Schutzgruppen erhalten bleiben, so verwendet man eine geeignete Kombination der Synthese-und Abspaltmethoden.

Für die Synthese von Peptiden mit einer C-terminalen Amidgruppierung oder einer ω-Amino- bzw. ω-Guanidinoalkylgrup pierung wird ebenfalls das von Sheppard beschriebene modifizierte Trägerharz verwendet. Nach der Synthese wird das in der Seitenkette voll geschützte Peptid vom Harz abgespalten und anschließend in klassischer Lösungssynthese mit dem entsprechenden Amin bzw. ω-Aminoalkylamin oder ω-Guanidinoalkylamin umgesetzt, wobei gegebenenfalls vorhandene weitere funktionelle Gruppen in bekannter Weise temporär geschützt werden können.

Die folgenden Beispiele dienen zur Erläuterung der Erfindung, ohne daß diese darauf beschränkt wäre.

A.Herstellung der OObt-Ester

Beispiel 1:

Fmoc-Ile-OObt

50 mMol Fmoc-Ile-OH werden zusammen mit 50 mMol HOObt in 100 ml trockenem THF gelöst oder suspendiert und bei 0°C 55 mMol Dicyclohexylcarbodiimid eingetragen. Man läßt über Nacht reagieren, saugt dann vom ausgefallenen Dicyclohexylharnstoff ab und entfernt das Lösungsmittel im Vakuum. Der Rückstand wird in Methylenchlorid gelöst und im Eisbad abgekühlt. Dabei fällt noch weiterer Harnstoff aus. Man filtriert erneut und entfernt das Lösungsmittel zuletzt im Hochvakuum. Der Rückstand wird mit Petrolether verrieben, abgesaugt und im Hochvakuum getrocknet.

Der erhaltene Aktivester wird mittels DC (Kieselgel mit Essigester als Laufmittel) und HPLC ((R)Vydak C$_{18}$-Säule mit einem Gradienten von 40 % A [80 % Acetonitril/Wasser mit 0,1 % Trifluoressigsäure ] auf 90 % A in 0,1 % wäßriger Trifluoressigsäure innerhalb 30 Minuten) auf ihre Reinheit überprüft.
Ausbeute: 15,5 g, $[\alpha]_D^{20}$ = -93,5° (c = 1, DMF)

In analoger Weise wurden folgende Aktivester erhalten:

Beispiel 2

Fmoc-Leu-OObt

$[\alpha]_D^{20}$ = -127,2° (c = 1, DMF)

Beispiel 3:

Fmoc-Asp(OtBu)-OObt

$[\alpha]_D^{20}$ = -61.5° (c = 1, DMF)

Beispiel 4:

Fmoc-Pro-OObt

Fp. 159-161°C (aus Diisopropylether und wenig Isopropanol)
$[\alpha]_D^{20}$ = -144,5° (c = 1, DMF)

Beispiel 5:

Fmoc-Glu(OtBu)-OObt

Fp. ab 95°C (Zersetzung; umkristallisiert aus Isopropanol)
$[\alpha]_D^{20}$ = -94,5° (c = 1, DMF)

Beispiel 6:

Fmoc-Gly-OObt

Fp. 149-151°C (aus Diisopropylether und wenig Isopropanol)

Beispiel 7:

Fmoc-Ala-OObt

Fp. 152-154°C, $[\alpha]^{20}$ =$_D$ -158° (c = 1, DMF)

Beispiel 8:

Fmoc-Cys(tBu)-OObt

Fp. ab 98° (Zersetzung, umkristallisiert aus CH$_2$Cl$_2$/Ether/Diisopropylether
$[\alpha]_D^{20}$ = -150,2° (c = 1, DMF)

Beispiel 9:

Fmoc-Val-OObt

Fp. 114-116°C, $[\alpha]^{20}$ = -136,9° (c = 1, DMF)

Beispiel 10:

Fmoc-Lys(Boc)-OObt

Fp. ab 150°C, Zersetzung (aus Essigester/Diisopropylether)
$[\alpha]_D^{20}$ = -37,1° (c = 1, DMF)

Beispiel 11:

Fmoc-Phe-OObt

Fp. 187-189°C (aus $CH_2Cl_2$/Ether),
$[\alpha]_D^{20}$ = -113,7° (c = 1, DMF)

Beispiel 12:

Fmoc-Tyr(tBu)-OObt

$[\alpha]_D^{20}$ = -85° (c = 1, DMF)

Beispiel 13:

Fmoc-Cys(4-MeOBzl)-OObt

Fp. 98-101°C (aus $CH_2Cl_2$/Petrolether),
$[\alpha]_D^{20}$ = -75,5° (c = 1, DMF)

B. Anwendung der HOOBt-Aktivester

B1 Lösungssynthese

Beispiel 14:

Fmoc-Leu-Arg-OH

In einer Mischung aus 1,74 g L-Arginin, 1,63 g HOObt und 1,79 g Pyridiniumperchlorat in 50 ml Dimethylformamid gibt man bei Raumtemperatur 5 g Fmoc-Leu-OObt. Man rührt 3 Stunden bei Raumtemperatur und läßt über Nacht bei Raumtemperatur stehen. Die klare Lösung wird eingeengt und der Rückstand mit Wasser versetzt. Mit wäßriger $NaHCO_3$-Lösung wird ein pH von 7 eingestellt. Der Niederschlag wird abgesaugt, mit Wasser gewaschen und getrocknet.
Ausbeute: 4,7 g, Fp. 145-155°C (unter Zersetzung), $[\alpha]_D^{23}$ = -18,5° (c = 1, Methanol)

B2 Festphasensynthese

Beispiel 15:

H-Val-Gln-Ala-Ala-Ile-Asp-Tyr-Ile-Asn-Gly-OH

Die Synthese des obigen Decapeptids (ACP 65-74) erfolgte an einem mit Glycin beladenen Pam-Harz der Fa. Applied Biosystems unter Verwendung der erfindungsgemäßen HOObt-Ester. Die Synthese wurde mit einem automatischen Peptid-Synthesizer Modell 430A der Fa. Applied Biosystems durchgeführt. Dazu wurde jeweils 1 mMol Fmoc-Val-OObt, Fmoc-Ala-OObt, Fmoc-Ile-OObt, Fmoc-Asp(OtBu)-OObt und Fmoc-Tyr(tBu)-OObt in die vom Hersteller gelieferten Cartridges eingewogen. Fmoc-Gln-OH und Fmoc-Asn-OH wurden zusammen mit 1,5 mMol HOBT in diese Cartridges eingewogen. Die Voraktivierung dieser Aminosäuren erfolgte direkt in den Cartridges durch lösen in 4 ml DMF und Zugabe von 2 ml einer 0,55 M Lösung von Diisopropylcarbodiimid in DMF. Die HOObt-Ester wurden in 6 ml DMF gelöst und dann ebenso wie die in situ voraktivierten Aminosäuren Glutamin und Asparagin auf das vorher mit 20 % Piperidin in DMF entblockierte Harz gepumpt. Zur Synthese wurde 0,5 mMol Gly-Harz eingesetzt und Glutamin und Asparagin doppelt gekuppelt. Nach beendeter Synthese wurde das Peptid mit einer 10 : 1 Mischung von Trifluoressigsäure und Trifluormethansulfonsäure unter Zusatz von m-Kresol und Thioanisol als Kationenfänger vom Harz abgespalten und das Rohpeptid an (R)Sephadex G25 mit 10 %iger Essigsäure als Elutionsmittel entsalzt. Die peptidhaltigen Fraktionen wurden vereinigt und die Reinheit und Identität des Produktes mittels HPLC überprüft.

**Ansprüche**

1. Aminosäurederivat der Formel
X - A - OObt
in welcher
X eine Urethanschutzgruppe,
A Gly, Ala, Val, Leu, Ile, Cys(tBu), Cys(Acm), Met, Pro, Lys(Boc), Asp(OtBu), Glu(OtBu), Phe, Tyr-(tBu), Trp, Lys(Z), Lys(Bzl) oder Cys (4-MeOBzl), jeweils in ihrer L-oder ihrer D-Form
und
OObt 4-Oxo-3,4-dihydro-1,2,3-benzotriazin-3-yloxy
bedeuten.

2. Verfahren zur Herstellung einer Verbindung gemäß Anspruch 1, dadurch gekennzeichnet, daß man eine Verbindung der Formel X-A-OH, worin X und A wie im Anspruch 1 definiert sind, mit 3-Hydroxy-4-oxo-3,4-dihydro-1,2,3-benzotriazin (HOObt) in Gegenwart eines Carbodiimids umsetzt.

3. Verfahren zur Herstellung eines Peptids durch Fragmentkupplung, dadurch gekennzeichnet, daß man mindestens eine Verbindung gemäß Anspruch 1 als Kupplungskomponente verwendet.

4. Verfahren gemäß Anspruch 3, dadurch gekennzeichnet, daß ein Fragment während des Syntheseablaufs an einem Festkörper kovalent gebunden ist.

5. Verwendung einer Verbindung gemäß Anspruch 1 bei der Herstellung eines Peptids mittels Fragmentkupplung.

6. Verwendung einer Verbindung gemäß Anspruch 1 bei der Festkörpersynthese eines Peptids.

<u>Patentansprüche für die folgenden Vertragsstaaten : Spanien und Österreich:</u>

1. Verfahren zur Herstellung eines Aminosäurederivates der Formel
X - A - OObt
in welcher
X eine Urethanschutzgruppe,
A Gly, Ala, Val, Leu, Ile, Cys(tBu), Cys(Acm), Met, Pro, Lys(Boc), Asp(OtBu), Glu(OtBu), Phe, Tyr(tBu), Trp, Lys(Z), Lys(Bzl) oder Cys (4-MeOBzl), jeweils in ihrer L-oder ihrer D-Form
und
OObt 4-Oxo-3,4-dihydro-1,2,3-benzotriazin-3-yloxy
bedeuten, dadurch gekennzeichnet, daß man eine Verbindung der Formel X-A-OH, worin X und A wie oben definiert sind, mit 3-Hydroxy-4-oxo-3,4-dihydro-1,2,3-benzotriazin (HOObt) in Gegenwart eines Carbodiimids umsetzt.

2. Verfahren zur Herstellung eines Peptids durch Fragmentkupplung, dadurch gekennzeichnet, daß man mindestens eine Verbindung gemäß Anspruch 1 als Kupplungskomponente verwendet.

3. Verfahren gemäß Anspruch 3, dadurch gekennzeichnet, daß ein Fragment während des Syntheseablaufs an einem Festkörper kovalent gebunden ist.

4. Verwendung einer Verbindung gemäß Anspruch 1 bei der Herstellung eines Peptids mittels Fragmentkupplung.

5. Verwendung einer Verbindung gemäß Anspruch 1 bei der Festkörpersynthese eines Peptids.